# EUROPEAN PATENT APPLICATION

(11) **EP 0 646 384 A2**
(43) Date of publication of application: **05.04.1995**
(21) Application number: 94500144.4
(22) Date of filing: 10.08.1994
(51) Int. Cl.: A61M 5/50, A61M 5/32

(54) **Safety syringe**

(30) Priority: 26.08.1993 ES 9302341
(71) Applicant: Baucells Granell, Jaime, E-08025-Barcelona (ES)
(72) Inventor: Baucells Granell, Jaime, E-08025-Barcelona (ES)
(74) Representative: Gomez-Acebo y Pombo, José Miguel

(57) **Abstract**

Safety syringe comprising a cylindrical tubular chamber (1), a breakable plunger (2), breakable at a weakened section (9), and a syringe-needle holder (6) which is fixed in the front part (5,5') of the chamber (1) and which has, in its back side, a hollow section that cooperates with a shank (13) which axially protrudes from the plunger (2) in a manner as to fit into the hollow section of the syringe-needle holder (2) to retract same upon drawing the plunger (2) until the needle (7) is found completely inside the chamber (1).

## Description

The present invention refers to a safety syringe, of the type comprising a chamber with a tubular cylindrical configuration, a plunger within said chamber being able move along the length of the chamber, and a syringe-needle holder fixed to the end of the syringe opposite that of plunger.

More specifically, the syringe of the invention is of the type comprising a breakable plunger, which prevents the re-use of the syringe, and which provokes the retraction of the needle into the interior of the chamber, after the first forward or injection movement, thus preventing additional accidental pricks or injuries. To effect this, the front end of the plunger has a section that connects with the syringe-needle holder.

Within the area of the present invention, syringes with the section connecting with the holder comprising the forward section having end clamps capable of externally grasping the holder upon reaching the maximum penetration point in the chamber and drag said syringe-needle holder backward upon initiating the return or backward movement of the plunger.

Mentioned combination, although assuring a good performance, could result in increase manufacturing costs.

The present invention provides a safety syringe of type above indicated, having a retractable needle and breakable plunger, the plunger having a unit that connects with the syringe-needle holder, said unit having a very simple form, such that it allows considerably reducing manufacturing costs.

According to the present invention, the end of the plunger which connects with the syringe-needle holder comprises longitudinally ribbed shank, having the same size throughout its length and preferably greater than the depth of the syringe-needle holder. This shank protrudes coaxially from the injecting head or end of the plunger and has a maximum radius cross-section equal to, or slightly superior, than the internal redius of the syringe-needle holder. This shank's extreme preferably tapers in its configuration through a progresive reduction of the size of its ribs.

The shank may be composed of equal radial walls, with a star configuration, each wall's height being equal or slightly greater than the internal radius of the syringe-needle holder.

Based on the configuration explained, when the plunger advances, and upon almost reaching its maximum forward position, the shank commences its penetration into the syringe-needle holder. When the plunger reaches its maximum forward position, the shank will have reached its maximum penetration position within the syringe-needle holder. The introduction and penetration of this shank into the holder will require a slight increase in the pressure exerted on the back head of the plunger during its forward movement.

Once the forward movement of the plunger should have been completed, if said plunger is pulled backward, the shank will pull on the syringe-needle holder, thus pulling the needle inside the chamber. Once the needle should have been pulled totally into the chamber, the plunger may be broken, thus preventing the re-use of the syringe.

The shank of the plunger may have a section, close to the forward head of the plunger, having a greater cross-section, around which an elastically deformable mass may be introduced, which hermetically fits against the internal surface of the chamber, thus providing a perfect fit of forward head of the plunger within the chamber, thus guaranteeing the injection and suction actions of the syringe when in use.

The chamber of the syringe may be composed of two sections, the chamber itself, and a ferrule coupled to said chamber in its front end, thus allowing the coupling of the syringe-needle holder, or the chamber and ferrule being one single unit, then requiring that the syringe-needle holder be introduced from the back. In the later case, both the chamber where the holder is to be located, and syringe-needle holder, will have a slightly conical form.

The body of the syringe-needle holder is divided into two parts, the first, having a greater diameter, internally hollow, its exterior form being plain and frustroconic, and continuous in order to fit perfectly into its corresponding chamber and allowing an interpositioning of at least one thin external annular transverse ring, and the other, of a lesser diameter, axially perforated and having plane radial walls.

The above mentioned characteristics will be better understood with the following description, effected with reference to the enclosed drawings, in which possible variants of the invention are shown, these being given simply as examples and not to be understood as limited to these.

In the drawings:

Figure 1 is a longitudinal section of a syringe manufactured in accordance with the invention.

Figure 2 is a magnified section of the syringe, drawn as seen through II-II of Figure 1.

Figure 3 is a transverse magnified section view of the syringe, as seen through III-III of Figure 1.
Figure 4 is a partial longitudinal magnified section view, showing the front section of the syringe, with the plunger being in its most forward position.

Figure 5 is a section similar to that of Figure 4, showing a variant of the invention.

Figure 6 is a magnified transverse section of Figure 5, as seen from cut VI-VI.

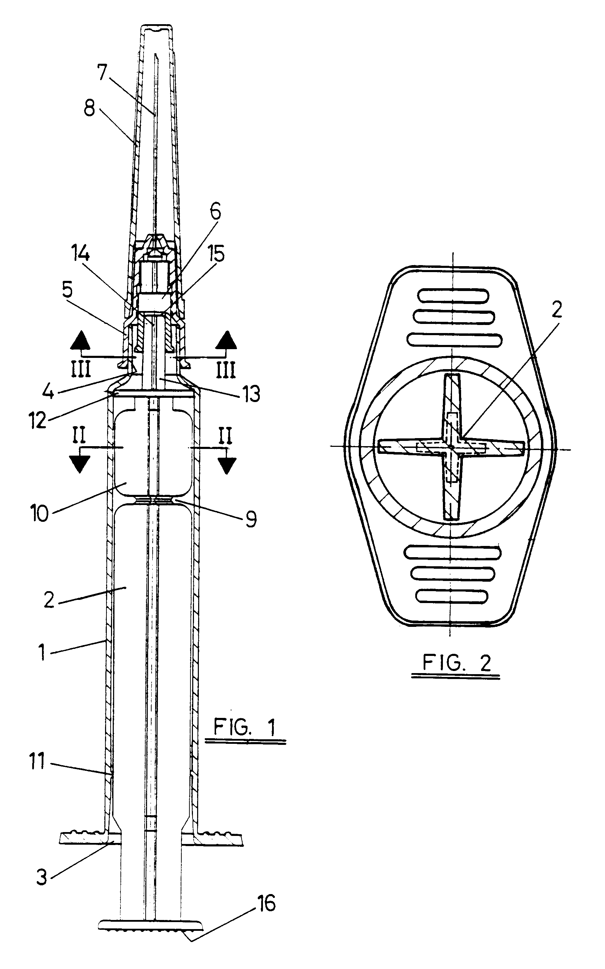

As may be appreciated in Figures 1, 2 and 3, the syringe comprises a cylindrical chamber 1, within which is introduced a plunger 2 capable of moving axially. Chamber 1 is open in its rear end 3, while its front end forms a mouth section 4 in which the ferrule 5, which carries the holder 6, is coupled, the holder 6 being one unit with the needle 7. A protective cap 8, to protect needle 7, may also be placed over ferrule 5, said cap 8 remaining in place until such time as the syringe is to be used.

Plunger 2, as seen in Figure 2, has a cross configuration cross-section, and has a weakened middle section 9, which will allow its being broken when the plunger is in its maximum retracted position, determined when the internal section 10 of the plunger hits the annular rib 11 which protrudes from the internal surface of the chamber 1. The breaking of the plunger will avoid its later penetration into the chamber 1, and thus prevent the re-use of the syringe.

Plunger 2 has, in its forward end, an impelling head 12, from which coaxially protrudes a shank 13 which is longitudinally ribbed. The shank 13 will shall be long enough to, upon the plunger 2 reaching its maximum penetration within the chamber 1, as represented in Figure 1, introduce itself within the syringe-needle holder 6.

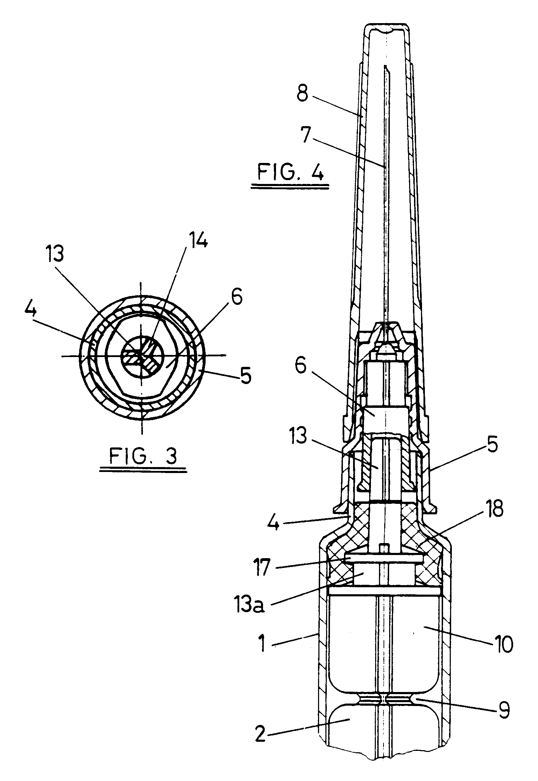

As seen in Figure 3, shank 13 may be formed by radial equal walls, defining a star-type cross-section format. Whatever be the format of shank 13, its circumference will have a maximum radius equal to, or slightly greater than, that of the interior of the syringe-needle holder 6. Additionally, the ribs or walls 14 will have rounded end sections 15, so that said shank 13 will have a decreasing end section, facilitating its introduction into the holder 6.

In accordance with the construction mentioned, when the plunger 2 moves forward, and shank 14 reaches holder 6, slightly more pressure will have to be exerted on the head 16 of the plunger 2 to provoke the introduction of shank 13 into holder 6, until the plunger head 12 shall have reached its maximum penetration limit, as shown in Figure 1.

Starting from the position shown in Figure 1, if the plunger 2 is retracted, or drawn backward, shank 13 would drag holder 6 along, and needle 7 with it, towards the interior of chamber 1. When internal section 10 of the plunger 2 rests on internal rib 11 of chamber 1, needle 7 will be totally within chamber 1. While in this same position, the weakened section 9 of plunger 2 will be situated outside the open base section 3, thus allowing an easy breaking of mentioned plunger 2 to prevent re-using the syringe.

As may be seen in Figure 4, shank 13 may have a back section 13a having a greater cross-section, ending in a peripheral wing 17, which would permit fixing the deformable elastic mass 18, which fits snug agains the internal surface of chamber 1, giving an air-tight closure between plunger 2 and said chamber 1.

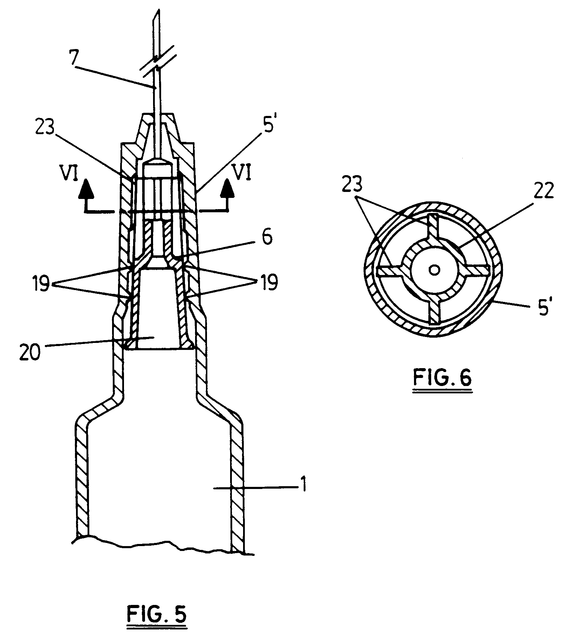

Figures 5 and 6 show a variant of the invention, in which the different sections have been given the same reference numbers.

Figure 5 shows that chamber 1 and ferrule 5, of Figures 1 and 4, are one single unit, and that the section corresponding to ferrule 5, designated as 5' and forming a smaller housing for holder 6, has a slight conical configuration and has annular lips 19 which allow a perfect coupling with the corresponding sectin of the syringe-needle holder 6.

Syringe-needle holder 6 generally has the same conical configuration as section 5' of the chamber and has two totally different sections, one designated by number 20, which is frustroconically hollow, and the other designated by number 21, which has a perforated nucleus 22 and radial walls 23 with decreasing heights in accordance with the conical configuration determined by section 20.

The priorly explained characteristics result in a safety syringe, having a simple constructions and reeuced costs, given that the section having to effect the retraction of needle 7 into the interior of chamber 1 is composed of a simple shank 13, which is easily manufactured.

## Claims

1. Safety syringe, of the type comprising a chamber (1), of tubular cylindrical configuration, a plunger (2) which may be broken at a weakened section (9), and a syringe-needle holder (6) fixed to the mouth-end section (4) of the chamber (1) by means of a tubular coupling, said plunger (2) having an impelling head (12) from which axially protrudes an end section or shank (13) which can connect with the holder (6) of the needle, characterized in that mentioned end section (13) which can connect with holder (6) of the needle (7) is composed of a longitunidally ribbed shank (13) which is long enough to penetrate at least partially inside the syringe-needle holder (6) when the plunger (2) is at its maximum inserted position, the shank (13) coaxially protruding from the impeller head (12), and has a maximum radial cross-section equal to, or slightly greater than, the internal radius of the syringe-needle holder (6), the shank (13) having one end (15) with a decreasing from given a progressive decrease in the height of the ribs.

2. Syringe according to claim 1, characterized in that the shank (13) is composed of equal radial walls (14), which give it a star-type format, each wall (14) having a height equal to, or slightly greater than, the internal radius of the syringe-needle holder (6).

3. Syringe according to claim 1, characterized in that the shank (13) has a section (13a), adjacent to the impelling head (17) of the plunger (2), of greater cross-section, around which is a packing mass (18) of deformable elastic material, which hermetically fits against the internal wall of the chamber (1), mentioned shank (13) having a length such as to protrude beyond mentioned mass (18) and the protruding length being sufficient to penetrate into the syringe-needle holder (6), at least partially, when the plunger (2) reaches its maximum penetration position inside the cylindrical tubular chamber (1).

4. Syringe according to claim 1, characterized in that the chamber (1) is one single unit and has, at its front end, opposite the end where the plunger (2) is introduced, a tapered housing or ferrule (5') for the holder (6), both the ferrule (5') and the holder (6) being slightly conical.

5. Syringe according to claim 4, characterized in that the ferrule (5') is provided with annular lips (19).

6. Syringe according to claim 4, characterized in that the syringe-needle holder (6) has two sections, one being frustroconic and hollow (20), and the other a perforated nucleus (22) and radial walls (23) having decreasing heights in accordance with the conical form of the section (20).
